# EUROPEAN PATENT SPECIFICATION

(11) **EP 3 436 830 B1**
(45) Date of publication and mention of the grant of the patent: **02.12.2020**
(21) Application number: 17714199.1
(22) Date of filing: 28.03.2017
(51) Int. Cl.: G01N 33/574

(54) **THSD7A AS A NOVEL TARGET FOR CANCER THERAPY AND DIAGNOSIS**
THSD7A ALS NEUES TARGET FÜR DIE KREBSTHERAPIE UND KREBSDIAGNOSE
THSD7A COMME NOUVELLE CIBLE POUR LE TRAITEMENT ET LE DIAGNOSTIC DU CANCER

(30) Priority: 29.03.2016 EP 16162545
(43) Date of publication of application: 06.02.2019
(73) Proprietor: Universitätsklinikum Hamburg-Eppendorf, 20246 Hamburg (DE)
(72) Inventor: STAHL, Rolf, 22359 Hamburg (DE); HOXHA, Elion, 22453 Hamburg (DE); STAHL, Phillip, 20251 Hamburg (DE); WIECH, Thorsten, 20255 Hamburg (DE)
(74) Representative: Uexküll & Stolberg
(86) International application number: PCT/EP2017/057347
(87) International publication number: WO 2017/167770

(56) References cited:
- US-A1- 2015 284 808
- KELLY J SOADY ET AL: "Mouse mammary stem cells express prognostic markers for triple-negative breast cancer", BREAST CANCER RESEARCH, CURRENT MEDICINE GROUP LTD, GB, vol. 17, no. 1, 4 March 2015 (2015-03-04), page 31, XP021220586, ISSN: 1465-5411, DOI: 10.1186/S13058-015-0539-6
- DUCRAY FRANÃ PRG OIS ET AL: "An ANOCEF genomic and transcriptomic microarray study of the response to radiotherapy or to alkylating first-line chemotherapy in glioblastoma patients", MOLECULAR CANCER, BIOMED CENTRAL, LONDON, GB, vol. 9, no. 1, 7 September 2010 (2010-09-07), page 234, XP021077972, ISSN: 1476-4598, DOI: 10.1186/1476-4598-9-234
- NICOLA M TOMAS ET AL: "Thrombospondin Type-1 Domain-Containing 7A in Idiopathic Membranous Nephropathy", NEW ENGLAND JOURNAL OF MEDICINE, THE - NEJM -, MASSACHUSETTS MEDICAL SOCIETY, vol. 371, no. 24, 11 December 2014 (2014-12-11), pages 2277-2287, XP009181856, ISSN: 0028-4793

## Description

The invention relates to a novel marker protein that is expressed by tumor cells. The marker protein Thrombospondin Type-1 Domain-containing 7A (THSD7A) can be used for diagnosing a tumor in a subject by detecting antibodies against this marker protein in the blood of the patient.

The invention also provides a method of screening drug candidates to identify a drug that is useful for the treatment of cancer which method involves determination of the THSD7A expression level. Finally, the invention provides novel therapeutic approaches for treating THSD7A-expressing cancers.

### FIELD OF THE INVENTION

The small blood vessels in the kidney, i.e. the glomeruli are responsible for filtering waste from the blood. Membranous nephropathy is an autoimmune disease which leads to inflammation in the glomeruli and thickening of their capillary walls. The disease is caused by the deposition of immune complexes on the subepithelial side of the glomerular basement membrane. As a result from the inflammation, proteins leak from the blood vessels into the urine, a condition known as proteinuria. The leakage involves the risk of long-term kidney damage, and kidney failure is often the ultimate clinical outcome.

It has been suggested in the literature that there may be an association between membranous nephropathy and malignant tumors (Beck & Salant (2014), J Clin Invest, 124:2307-14). It has been suspected that the immune system massively produces antibodies in response to the tumor which leads to membranous nephropathy. However, a tumor antigen was not identified so that the hypothesis is solely based on the clinical association of the two diseases.

In the course of the present invention, it was found that the protein Thrombospondin Type-1 Domain-containing 7A (THSD7A) in many cases appears to be the target antigen on tumor cells which induces the production of antibodies that leads to membranous nephropathy.

Soady KJ et al. (2015), Breast Cancer Research, vol. 17, 31, and Ducray F et al. (2010), Molecular Cancer, vol. 9, 234 disclose that THSD7A is overexpressed in certain cancer types. However, none of these references discloses the measurement of THSD7A in the blood.

### DESCRIPTION OF THE INVENTION

The insight that THSD7A is expressed on tumors allows the provision of diagnostic and prognostic methods that measure the expression of THSD7A in a tissue of a patient, or the presence of antibodies against THSD7A in a blood sample of a patient.

Therefore, in a first aspect, the invention relates to a method for determining whether a subject is afflicted with cancer or is susceptible to developing cancer, comprising
a) providing a blood sample from a subject; and
b) determining the presence of antibodies against a Thrombospondin type-1 domain-containing 7A (THSD7A) protein in said blood sample of said subject;
wherein the presence of antibodies against a THSD7A protein indicates that said subject is afflicted with cancer or is susceptible to developing cancer.

The method of the invention is conducted with a blood sample that can be obtained from the subject to be tested by commonly known measures, such as venipuncture. Preferably, the subject to be tested is a human subject. However, the method is not limited to humans, but can instead be used for mammalian animals. The blood sample can be a whole-blood sample, or it can be a serum or plasma sample derived from a whole-blood sample. The antibodies to be detected in step (b) of the above method are directed against a THSD7A protein, preferably against a human THSD7A protein. In a particularly preferred embodiment of the invention, the antibodies to be detected are directed against an epitope which is located within the sequence of SEQ ID NO:1. The sequence set out in SEQ ID NO:1 is the amino acid sequence of the human THSD7A protein. The sequence set out in SEQ ID NO:2 is the corresponding genomic sequence that codes for the human THSD7A protein.

Alternatively, the antibodies to be detected in step (b) of the above method may be directed against a THSD7A protein variant which has at least 90% sequence identity to SEQ ID NO:1. As will be appreciated by the person of skill, the THSD7A protein that naturally occurs in humans might differ to some extent between different individuals due to mutations. As a consequence, THSD7A proteins may occur which differ in one or more amino acids from the amino acid sequence in SEQ ID NO:1. These variants of THSD7A are encompassed by the present invention. Hence, as used herein, variants of the protein of SEQ ID NO:1 are proteins with an amino acid sequence that differs from the amino acid sequence of the protein of SEQ ID NO:1 by one or more amino acid substitutions, additions or deletions. In general, any amino acid residue of the sequence shown in SEQ ID NO:1 may be substituted by another amino acid, provided that the resulting protein variant still exhibits an THSD7A activity. Determination of the THSD7A activity can be performed, for example, by measuring the activation of downstream targets of THSD7A. For example, phosphorylation of focal adhesion kinase (FAK) downstream of THSD7A activation can be determined by western blot. In particular, the invention encompasses protein variants which differ from the amino acid sequence shown in SEQ ID NO:1 in up to 5, 10, 15, 20 or even up to 25 amino acid positions.

It is preferred that the substitutions are conservative substitutions, i.e. substitutions of an amino acid residue by another amino acid of a similar polarity, which acts as a functional equivalent. Preferably, the amino acid residue used as a substitute is selected from the same group of amino acids as the amino acid residue to be substituted. For example, a hydrophobic residue can be substituted with another hydrophobic residue, or a polar residue can be substituted with another polar residue having the same charge. Functionally homologous amino acids which may be used for a conservative substitution comprise, for example, non-polar amino acids such as glycine, valine, alanine, isoleucine, leucine, methionine, proline, phenylalanine, and tryptophan. Examples of uncharged polar amino acids comprise serine, threonine, glutamine, asparagine, tyrosine and cysteine. Examples of charged polar (basic) amino acids comprise histidine, arginine and lysine. Examples of charged polar (acidic) amino acids comprise aspartic acid and glutamic acid.

Also comprised by the term "variant" are protein sequences which include more amino acids than the sequence of SEQ ID NO:1, i.e. protein sequences in which one or more amino acids have been inserted. Such insertions may in principle occur at any position of the protein of SEQ ID NO:1. The insertions may be stretches of contiguous amino acids comprising, for example, 2, 3, 4, 5, 6, 7, 8, 9 or ten amino acids. Similarly, the term "variant" also includes protein sequences in which one or more amino acids are deleted as compared to the protein of SEQ ID NO:1. The deletion can involve several contiguous amino acid residues of the protein of SEQ ID NO:1, for example, 2, 3, 4, 5, 6, 7, 8, 9 or ten amino acids. Variants of the protein of SEQ ID NO:1 may also include structural modifications, for example modified amino acids, such as amino acids that have been altered by phosphorylation, glycosylation, acetylation, thiolation, branching and/or cyclization.

The variants of the THSD7A protein set out in SEQ ID NO:1, which have been modified by substitution, addition or deletion of amino acids, normally show a considerable degree of overall amino acid sequence homology or sequence identity to the THSD7A protein of SEQ ID NO:1. Preferably, the homology or identity on the amino acid level between the variant and the THSD7A protein of SEQ ID NO:1 is at least 70%, 80%, 90%, 91%, 92%, 93%, 94%, 95%, 96%, 97%, 98% or 99% if the sequence of the variant is optimally aligned with that of SEQ ID NO:1. Methods and computer programs for determining amino acid homology are well known in the art.

The antibodies may also be directed to a fragment of the THSD7A protein of SEQ ID NO:1 or a fragment of a THSD7A protein variant as described above. This means that the antibodies to be detected are directed to a truncated form of the THSD7A protein.

Different techniques can be used for detecting the antibodies. For example, magnetic beads can be used which comprise recombinantly expressed THSD7A protein on their surface. Antibodies from the blood sample which have bound to the magnetic beads can be visualized by a colorimetric reaction or the like. Other suitable methods for detecting antibodies include immunoblotting, immunofluorescence tests, latex agglutination, enzyme-linked immunosorbent assay, and microparticle enzyme immunosorbent assay. Suitable techniques for qualitatively or quantitatively detecting specific antibodies in blood, plasma or serum have been described in the prior art and moreover belong to the common knowledge of a skilled person.

Where the blood of the subject comprises antibodies against a THSD7A protein, this indicates that said subject is either already afflicted with a cancerous disease or has an increased risk of developing such disease. Thus, in the event that the blood sample contains THSD7A antibodies, the patient should be thoroughly analyzed for the presence of a solid tumor or other forms of cancer, e.g. by scanning the patient's body in a CT scan for tumors. The tumor can be, but is not limited to, a kidney, prostate, urinary bladder, thyroid, esophagus, colon, breast, or lung tumor. In addition, the detection of THSD7A antibodies in the blood of the patient might be indicative for a hematologic cancer, such as leukemia, lymphoma, and multiple myeloma. Where the screening does not result in the identification of a cancerous disease, the patients should be subjected to early detection examination on a regular basis, as these patients have an increased risk of developing a tumor disease.

In a particularly preferred embodiment of the invention, the subject to be tested by the above method is afflicted with membranous nephropathy. In this case, the antibodies produced by the patient's immune system against the THSD7A protein are causative for the membranous nephropathy.

In a second aspect, the invention provides a method of screening drug candidates to identify a drug that is useful for the treatment of cancer, comprising:
a) providing a cell that expresses a THSD7A protein, preferably the sequence of SEQ ID NO:1 or a sequence having at least 90% sequence identity thereto or a fragment of any of these sequences;
b) adding a drug candidate to said cell; and
c) determining the effect of said drug candidate on the expression of said sequence or sequence fragment or on growth, survival, or motility of the cell;
wherein the reduction of expression by the cell or the reduction of growth, survival or motility of the cell indicates that the drug candidate may be useful as a drug for the treatment of cancer.

The screening method of the invention uses in step (a) a cell that expresses a THSD7A protein. The cell to be used in the method of the invention can be any cell that naturally expresses a THSD7A protein, such as a human tumor or non-tumor cell. Alternatively, the cell can be a genetically engineered cell that has been modified by the introduction of an expression vector or another element that provides for the heterologous expression of the THSD7A protein. The engineered cell may be a human or non-human cell, such as a mammalian cell, a bacterial cell, a yeast cell or an insect cell.

In a preferred aspect, the THSD7A protein is expressed in a non-human mammalian cell, e.g. a mouse, rat, hamster, or non-human primate cell. Non-human mammalian cells which have been proven useful for the heterologous expression of polypeptides, preferably human polypeptides, include primary cells and tissues (e.g. murine bone marrow cells, haematopoietic stem cells, murine lymphocytes, muscle tissue of different species, hepatocytes, and the like) as well as cultured cell lines, e.g. Chinese hamster ovary (CHO) cells, monkey kidney cells (COS), baby hamster kidney (BHK) cells, immortalized murine blood lymphocytes and the like.

Mammalian expression systems and appropriate expression constructs are widely known in the art. The expression construct can be, for example, a viral or non-viral expression vector which is useful for introducing the THSD7A-encoding polynucleotides into a cell for subsequent expression of THSD7A. The expression vector can be an episomal vector, i.e. one that is capable of self-replicating autonomously within the host cell, or an integrating vector, i.e. one which stably incorporates into the genome of the cell.

An expression vector will normally comprise a promoter which is functionally linked to the polynucleotide which encodes the THSD7A. Suitable promoters include, but are not limited to, the cytomegalovirus promoter (CMV), the Spleen Focus Forming Virus (SFFV) U3 promoter and the adenoviral major late promoter (Ad MLP). As an optional component, the mammalian expression vector may include a suitable enhancer element for increasing the expression level. Examples include the SV40 early gene enhancer (Dijkema et al. (1985) EMBO J. 4: 761) and the enhancer of the long terminal repeat (LTR) of Rous Sarcoma Virus (Gorman et al. (1982b) Proc. Natl. Acad. Sci. 79: 6777). The expression vector also optionally comprises transcription termination sequences and polyadenylation sequences for improved expression of THSD7A. Suitable transcription terminator and polyadenylation signals can, for example, be derived from SV40 (Sambrook et al (1989), Molecular Cloning: A Laboratory Manual).

In a particularly preferred aspect, the expression vector is a viral expression vector. Viral vectors for use in the present invention typically comprise a viral genome in which a portion of the native sequence has been deleted in order to introduce a heterogeneous polynucleotide without destroying the infectivity of the virus. Due to the specific interaction between virus components and host cell receptors, viral vectors are highly suitable for efficient transfer of genes into target cells. Suitable viral vectors for facilitating gene transfer into a mammalian cell are well known in the art and can be derived from different types of virus, for example, from a retrovirus, adenovirus, adeno-associated virus (AAV), ortho-myxovirus, paramyxovirus, papovavirus, picornavirus, or al-phavirus.

Alternatively, non-viral vector systems may also be used for introducing the THSD7A-encoding polynucleotide. Non-viral vector systems typically involve non-viral expression vectors which are introduced into the cell, e.g. by liposome-mediated transfer, direct injection of naked DNA, or the gene gun technology.

Several mammalian expression systems are offered by different manufacturers and can be employed in the present invention for expression of THSD7A, such as plasmid- or viral vector based systems, e.g. LENTI-Smart™ (InvivoGen), GenScript® Expression vectors, pAdVAntage™ (Promega), ViraPower™ Lentiviral and Adenoviral Expression Systems (Invitrogen).

The THSD7A protein which is expressed by the cell preferably is a protein of human origin. More preferably, it comprises or consists of the sequence of SEQ ID NO:1. Alternatively, the cell may express a THSD7A protein that is a variant of the sequence of SEQ ID NO:1 as defined elsewhere herein, preferably a variant having a sequence with at least 90% sequence identity to the sequence of SEQ ID NO:1. The cell may also express a fragment of the THSD7A protein of SEQ ID NO:1 or a fragment of one of its variants. Variants and fragments of the THSD7A protein have been defined elsewhere herein.

The screening method of the invention further comprises the addition of a drug candidate to the cell that expresses the THSD7A protein. After contacting the cell with the drug candidate, the effect, if any, of said drug candidate on the cell, in particular the expression of the THSD7A protein or protein fragment is measured. A reduction of expression in cells that were contacted with the drug candidate relative to untreated cells indicates that the drug candidate may be useful as a drug for the treatment of cancer. These drug candidates should be subjected to further examination. Alternatively or in addition, the effect of said drug candidate on growth, survival, or motility of the THSD7A-expressing cell can be determined, and a reduction of growth, survival or motility of the THSD7A-expressing cell indicates that the drug candidate may be useful as a drug for the treatment of cancer. The screening method can be designed as a high-throughput method which allows the testing of several thousand candidate drugs within a short period of time. It is preferred that the reduction of expression is at least 2-fold, preferably at least 5-fold or at least 10-fold.

The drug candidate compounds used in the screening methods of the present invention can include all different types of organic or inorganic molecules, including peptides, oligo- or polysaccharides, fatty acids, steroids, and the like. Typically, the candidate compounds will be small molecules with less than about 2,500 daltons, less than 2000 daltons, less than 1500 daltons, less than 1000 daltons, or less than 500 daltons. Candidate compounds for use in screening methods can be provided in the form of libraries which comprise a high number of synthetic or natural compounds.

In still another aspect, the invention pertains to the use of an antibody, antibody fragment, nanobody or nanobody fragment that selectively binds to a THSD7A protein for the in vitro diagnosis of cancer.

The detection of a THSD7A protein, such as the protein of SEQ ID NO:1 or a variant sequence having at least 90% sequence identity thereto or a fragment of any of these sequences, on the surface of cancer cells can also be used for differentiating between different tumor types, e.g. in differential diagnosis. For example, most of the clear cell renal carcinomas are negative for THSD7A, while most of the chromophobe renal carcinoma express THSD7A. Thus, THSD7A expression can be reliably used for distinguishing these two types of renal carcinoma from each other. Thus in another aspect, the invention refers to a method of distinguishing certain tumor types from each other, preferably to a method of distinguishing clear cell renal carcinoma from chromophobe renal carcinoma by detecting the expression of THSD7A. In yet another aspect, the invention pertains to the use of a compound that selectively binds to a THSD7A protein, preferably to the human THSD7A protein of SEQ ID NO:1 or a fragment thereof, for use in tumor differential diagnosis.

### DESCRIPTION OF THE FIGURES

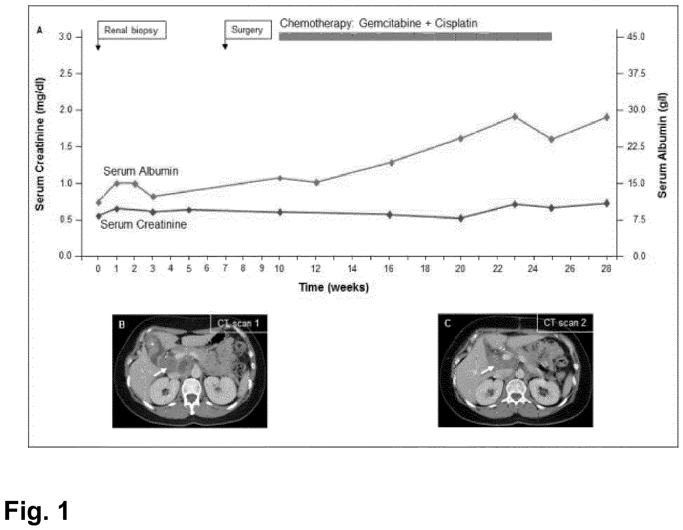

Figure 1 shows the serum creatinine and serum albumin levels and CT scans of the tumor in patient A. Serum creatinine remained within normal values during the follow-up. Serum albumin increased from 11 g/l at the time when MN was diagnosed to 29 g/l eighteen weeks after start of chemotherapy. Panel B: The CT-scan three weeks after diagnosis of MN shows a carcinoma in the gallbladder (*) and enlarged lymph nodes (arrow). Panel C: Following surgery and upon start of chemotherapy, the lymph node metastases showed no progression (arrow).

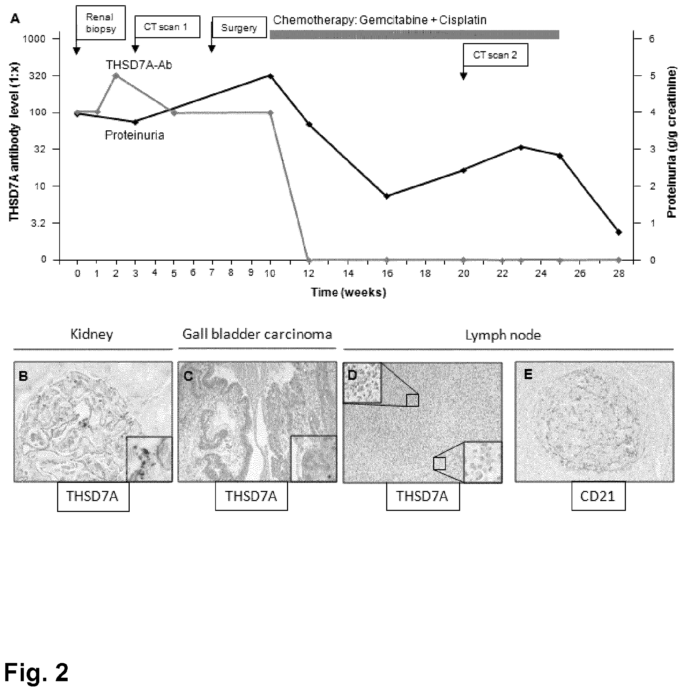

Figure 2 shows serum THSD7A antibody levels and proteinuria over time and morphologic characteristics of the patient.

Panel A: THSD7A antibody levels and proteinuria were elevated at the time of diagnosis of MN. Two weeks after start of chemotherapy with Gemcitabine and Cisplatin, THSD7A antibody levels fell followed by a decrease in proteinuria. At 28 weeks THSD7A antibody levels were not detectable and proteinuria was reduced to 0.7 grams/gram creatinine. Panel B: Immunohistochemical analysis of the renal biopsy showed a strongly enhanced glomerular THSD7A staining. Panel C: Mixed adenoneuro-endocrine carcinoma (MANEC) of the gallbladder. The carcinoma cells were positive for THSD7A in immunohistochemical analyses (right half) while dysplastic gall bladder cells showed a markedly lower positivity for THSD7A (left half). Panel D: Immunohistochemical analyses of lymph nodes infiltrated by metastases of the gall bladder carcinoma showed that the metastatic cells (lower right) as well as the follicular dendritic cells (upper left) stained positive for THSD7A. Panel E: Immunohistochemical analysis showing the CD21 expressing cells of the lymph node infiltrated by metastases of the gall bladder carcinoma, confirming that these are follicular dendritic cells.

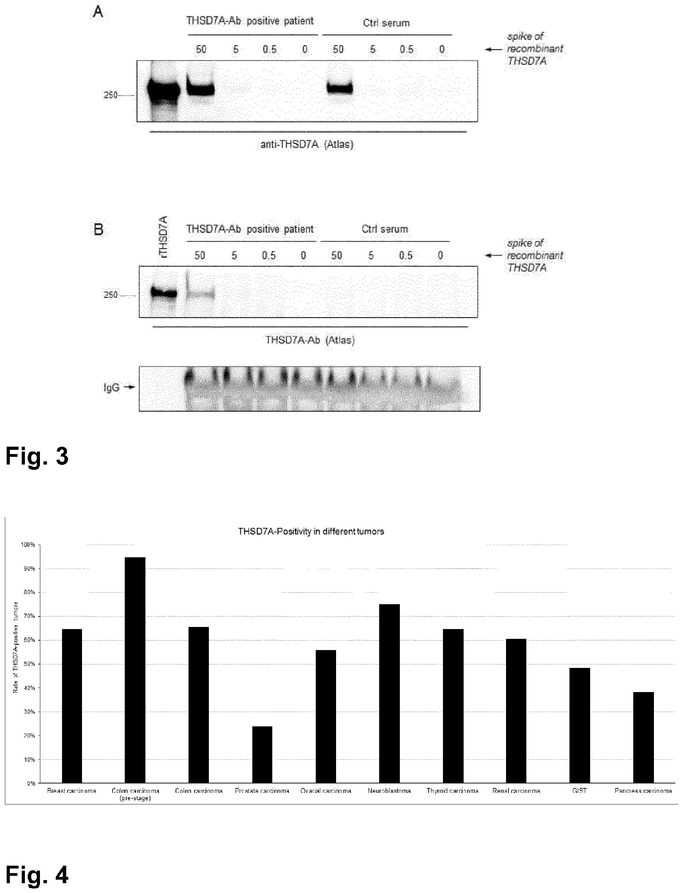

Figure 3 shows the result of the analysis of circulating THSD7A-THSD7A antibody immune complexes. Panel A: The numbering above lanes indicates the total protein amount of THSD7A-transfected HEK293 detergent-solubilized membrane extract used for each assay (50, 5 and 0.5 µg of total protein/assay) as a positive control to ensure that THSD7A could bind WGA beads and be detected by Western blot. No band was seen in the condition without spiked THSD7A while we could detect the recombinant THSD7A protein when spiking with 5 µg of total protein. Panel B: A faint band could be seen when only 5 µg of total protein from THSD7A transfected cells was added to the THSD7A antibody positive serum. However, no band was seen in serum without spiking, or when the THSD7A antibody positive serum is replaced by serum from a healthy donor. THSD7A-Ab: THSD7A antibodies; ctrl. serum: control serum. The analysis was performed as described in Tomas et al (2014), N Engl J Med, 371:2277-87.

Figure 4 shows the proportion of THSD7A-positive tumors in different malignant diseases. It can be seen that in some tumor types, the proportion of THSD7A-positive tumors is up to 70% (see, e.g. neuroblastoma).

### EXAMPLES

The following example describes the examination of a 40-year old woman, designated "Patient A" hereinafter, who was diagnosed with membranous nephropathy (MN). Upon closer examination, antibodies against THSD7A were found in the blood of the patient. A gallbladder tumor was discovered, and said tumor as well as corresponding lymph node metastases stained positive for THSD7A.

### Example 1: Clinical history and management of the patient

Patient A presented due to lower limbs edema and weight gain in the past two months. Her clinical history was unremarkable except for uterine myomas, diagnosed five years ago and hypothyroidism of unknown etiology, which was treated with thyroid hormones. An echocardiogram showed no abnormalities and the possibility of deep venous thrombosis was ruled out by sonography. Serum creatinine and urea levels were normal. Urinalysis showed proteinuria of 4.0 g/g creatinine with no hemoglobulinuria (see Figure 1A). Serum levels of C3, C4, carcinoembryonic antigen and cancer antigen 19-9 showed no abnormalities. Double-stranded-DNA, anti-nuclear antibodies and serologies for hepatitis A, B and C were negative. A gynecological examination revealed no abnormalities except for the myomas. Serum albumin was decreased to 11 g/l, while serum cholesterol and triglycerides were elevated to 294 mg/dl and 194 mg/dl, respectively.

### Example 2: Diagnosis of THSD7A-associated MN

MN was diagnosed in the patient by renal biopsy. A thickening of the glomerular capillary walls, a granular positivity for IgG in the immunohistochemistry and subepithelial electron dense deposits determined the diagnosis of membranous nephropathy.

### Example 3: Screening for THSD7A-antibodies in the blood

The blood of Patient A was screened for antibodies against THSD7A and PLA₂R1 by Western-Blot and immunofluorescence analysis as described in Tomas et al (2014), N Engl J Med, 371:2277-87.

Result: It was found that anti-THSD7A antibodies occurred in the blood of the patient. In contrast, no PLA₂R1-Ab could be detected in the plasma by indirect immunofluorescence, ELISA or Western blot.

### Example 4: Assay for the detection of circulating THSD7A immune complexes

In order to detect a potential circulating soluble form of THSD7A as well as THSD7A-THSD7A antibody immune complexes in the serum of the patient two different assays were performed as described in Tomas et al (2014), N Engl J Med, 371:2277-87. Briefly, for the detection of a circulating soluble form of THSD7A, 50 µl of THSD7A antibody positive patient or normal control serum were diluted 1:5 with 20 mM Tris pH 7.4, 140 mM NaCl, 1 mM CaCl₂ and mixed with wheat germ agglutinin (WGA) beads (Vector). As a positive control to ensure that THSD7A could bind WGA beads in the presence of other serum glycoproteins and can further be detected by Western blot, we used a detergent-solubilized fraction of recombinant THSD7A protein obtained from transfected HEK293 cells. THSD7A was spiked in the different sera in different amounts (50, 5 and 0.5 µg of HEK293 total membrane proteins) to determine the sensitivity of the assay. Samples were incubated overnight at 4°C. WGA beads were then collected by centrifugation, vigorously washed four times with 400 µl of 20 mM Tris pH 7.4, 140 mM NaCl, 1 mM CaCl₂, mixed with reducing Laemmli buffer, and boiled for 10 minutes. Samples were centrifuged and the supernatant was loaded on SDS-PAGE gels, electrophoresed, transferred to PVDF membranes, and detected with THSD7A antibody (Atlas) using high sensitivity ECL detection (Thermo Scientific).

In the second assay to detect circulating immune complexes consisting of soluble THSD7A and THSD7A antibodies, 50 µl of serum from the THSD7A antibody positive patient or serum from a healthy donor were diluted 1:5 with 20 mM Tris pH 7.4, 140 mM NaCl, 1 mM CaCl₂ and mixed with 30 µl of Protein G Sepharose (GenScript). As a positive control to ensure that the assay is able to detect THSD7A-THSD7A antibody immune complexes, sera were spiked with small amounts of solubilized recombinant THSD7A as described above. Samples were incubated overnight at 4°C. Protein G beads were then collected by centrifugation, vigorously washed four times with 400 µl of 20 mM Tris pH 7.4, 140 mM NaCl, 1 mM CaCl₂, mixed with reducing Laemmli buffer, and boiled for 10 minutes. Samples were centrifuged and the supernatant was loaded on SDS-PAGE gels, electrophoresed, transferred to PVDF membranes, and detected with THSD7A antibody (Atlas) using high sensitivity ECL detection (Thermo Scientific). Membranes were then reprobed with anti-human IgG antibodies to demonstrate efficient pull-down of serum antibodies for all samples.

Results: No circulating THSD7A-THSD7A-Ab immune complexes were detectable in the serum (see Figure 3)

### Example 5: Immunohistochemical analysis of renal biopsy

To determine whether THSD7A is the target antigen and the antibodies detected in the blood might be causative for the MN, a renal tissue biopsy was examined by THSD7A and PLA₂R1 staining as follows. Slides with kidney tissue were deparaffinized, pre-treated in citrate buffer (pH 6.1) for 15 min at 120°C and cooled down in iced water (10 min). After rinsing in 100% ethanol, slides were incubated 10 min with normal serum (Vector S2000) followed by THSD7A antibody (Sigma, rabbit, 1:4000 in antibody diluent, Invitrogen) or PLA₂R1 antibody (Sigma, 1:4000) over night at 4°C. The slides were then washed in PBS and incubated with polymer 1 (Zytomed Zytochem-Plus AP Polymer-Kit POLAP), rinsed in PBS and incubated with polymer 2 (Zytomed Zytochem-Plus AP Polymer-Kit POLAP). After washing in PBS, slides were stained in new fuchsin naphthol As-Bi phosphate substrate mixture (10 min) followed by 1 min nuclear staining in hemalaun (Mayer).

Result: The analysis revealed an increased immunohistological staining of THSD7A in the kidney of the patient (see Figure 2B). Based on the findings of Examples 1-5, it was concluded that the patient suffers from a THSD7A-associated form of MN.

### Example 6: Diagnosis of a gallbladder tumor

Three weeks after renal biopsy and diagnosis of MN, the patient presented again because of discomfort in the upper right abdomen. A computed tomogram (CT) scan showed a tumor in the gallbladder and enlarged lymph nodes around the portal vein (see Figure 1B). The gallbladder and three lymph nodes from the upper margin of the pancreas were surgically removed. Histopathologic examination revealed a mixed adenoneuroendocrine carcinoma (MANEC) of the gallbladder with three corresponding lymph node metastases. The infiltrated lymph nodes were non-regional and therefore considered as distant metastases. The tumor classification was: G3, pT2, M1 (LYM), L1, V0, Pn0 - tumor-free resection margin.

### Example 7: Immunohistochemical analysis of tumor tissue

Tissue samples of the removed gallbladder and lymph nodes were analyzed by staining for THSD7A essentially as outlined in Example 5 above, with the exception that the THSD7A antibody was diluted 1:500 in antibody diluent rather than 1:4000.

Results: The primary gallbladder tumor cells were positive for THSD7A in the immunohistochemical analysis (see Figure 2C). Likewise, the metastatic cells as well as follicular dendritic cells stained positive for THSD7A (see Figure 2D). Follicular dendritic cells are unique stromal cells that present stored antigens to maturing B cells for high affinity antibody formation. The nature of these cells was confirmed by detection of CD21 expression (see Figure 2E).

### Example 8: RT-PCR analysis of the tumor tissue

Tissue samples of the removed gallbladder and lymph nodes were analyzed by RT-PCR analysis. RNA isolation was performed based on 10 µM paraffin-embedded tissue sections with the "High Pure FFPE RNA Microkit" (Roche, Mannheim, Germany) according to the manufacturer's recommendations. 10 µl total RNA of the different samples were reverse transcribed by 200 U "Revert Aid" reverse transcriptase (Thermo Fisher Scientific, Schwerte, Germany) using 100 ng of a random hexamer primer mix (Life Technologies, Darmstadt, Germany) for one hour at 42°C. Real time PCR was performed in a "Step One-Real-Time PCR-System" (Life Technologies, Darmstadt, Germany) using 1 µl human THSD7A-specific (GenBank Accession No: MN_015204) Taqman-probes contained in the kit (Thermo Fisher Scientific, Nr.: Hs 00324295_m1), 10 µl 2 x Fast Universal PCR Mastermix (both Life Technologies) and 8 µl cDNA. For normalization of different RNA/cDNA qualities, "SYBR-Green" based real-time PCR (Eurogentec, Köln, Germany) using 1:100 diluted cDNA and human 18S rRNA-specific primers was performed.

Results: RT-PCR analysis showed that THSD7A mRNA was detectable in the gallbladder carcinoma, but not in the normal tissue of the gallbladder. Metastases and follicular dendritic cells (fDC) of metastases-infiltrated lymph nodes were also positive for THSD7A. See Figure 2D and 2E.

### Example 9: FISH analysis

For FISH analysis, a commercial kit was utilized (Histology FISH Accessory KIT, DAKO). A Spectrum Green-labelled THSD7A-probe (Empire Genomics) was used together with a Spectrum Orange-labelled CEP7-probe (Vysis-Abbott). Before hybridization, sections were deparaffinized, air-dried, dehydrated and then denatured at 72°C for five minutes in a ThermoBrite (Vysis-Abbott). After overnight hybridization at 37°C in a ThermoBrite, slides were washed and counterstained with 10 µl DAPI (Vysis-Abbott) .

Results: In FISH analysis, a polysomy of chromosome 7 was recognized with six copies of the THSD7A gene and chromosome 7 centromere.

### Example 10: Chemotherapy

Patient A was subjected to 6 cycles of chemotherapy with gemcitabine and cisplatin. Briefly, the patient received 50 mg cisplatin and 2000 mg gemcitabine on day 1 and day 8 of each cycle.

Result: Two weeks after start of chemotherapy, antibodies against THSD7A were no longer detectable in the blood, and the patient remained negative throughout the follow-up period. Four weeks later proteinuria had decreased to 1.8 g/g creatinine. Eighteen weeks after commencement of chemotherapy proteinuria had decreased to 0.7 g/g creatinine while serum albumin increased to 29 g/l. See Figure 2A. At the same time, the lymph node metastases showed no progress remaining at a constant size. See Figure 1C.

### Example 11: Screening for further tumors expressing THSD7A

Over 3.000 paraffin embedded tissue samples of human cancers were analyzed for THSD7A expression as outlined above in Example 7. This analysis concluded several (over 50) malignant tumor entities as well as benign lesions. THSD7A expression analysis was performed with a commercial probe (pH: 7.8, dilution: 1:150).

Result: Positive staining was observed in a number of malignant tumors, including chromophobe and papillary renal cell carcinoma, prostate carcinoma, medullary thyroid carcinoma, esophagus carcinoma, and medullary mammary carcinoma. In addition, THSD7A expression was also found in benign tumors such as colon adenoma and renal oncocytoma.

### Example 12: Screening sera from patients with MN

Sera from 1009 additional patients with MN were analysed by indirect immunofluorescence for the presence of THSD7A-Ab. 25 patients were found to be THSD7A-Ab positive. In all these patients, the positivity for THSD7A-Ab in the blood was confirmed by western blot, as described in Tomas et al (2014), N Engl J Med, 371:2277-87.

Result: Seven of those 25 patients, including Patient A, had a malignant tumor. These findings support a causal relationship between the expression of THSD7A in a tumor and the development of membranous nephropathy, thus describing a potential mechanism for the association between malignancy and membranous nephropathy.

## Claims

1. Method for determining whether a subject is afflicted with cancer or is susceptible to developing cancer, comprising
a) providing a blood sample from a subject;
b) determining the presence of antibodies against a Thrombospondin Type-1 Domain-containing 7A (THSD7A) protein in said blood sample;
wherein the presence of antibodies against the THSD7A protein indicates that said subject is afflicted with cancer or is susceptible to developing cancer.

2. Method according to claim 1, wherein the THSD7A protein comprises the sequence of SEQ ID NO:1 or a sequence having at least 90% sequence identity thereto or a fragment of any of these sequences.

3. Method according to claim 1 or 2, wherein the subject who is suspected to suffer from cancer is afflicted with membranous nephropathy.

4. Method according to claims 1-3, wherein said cancer is selected from, but not restricted to, kidney, prostate, thyroid, bladder, esophagus, colon or breast cancer.

5. A method of screening drug candidates to identify a drug that is useful for the treatment of cancer, comprising:
a) providing a cell that expresses a Thrombospondin Type-1 Domain-containing 7A (THSD7A) protein,
b) adding a drug candidate to said cell; and
c) determining the effect of said drug candidate on the expression of said sequence or sequence fragment or on growth, survival, or motility of the cell;
wherein the reduction of expression or reduction of growth, survival, or motility of the cell indicates that the drug candidate may be useful as a drug for the treatment of cancer.

6. Method according to claim 5, wherein the Thrombospondin Type-1 Domain-containing 7A (THSD7A) protein comprises or consists of the sequence of SEQ ID NO:1 or a sequence having at least 90% sequence identity thereto or a fragment of any of these sequences.

7. Use of an antibody, antibody fragment, nanobody or nanobody fragment that selectively binds to a Thrombospondin Type-1 Domain-containing 7A (THSD7A) protein for the in vitro diagnosis of cancer.

8. Use of an antibody, antibody fragment, nanobody or nanobody fragment of claim 7, wherein said Thrombospondin Type-1 Domain-containing 7A (THSD7A) protein comprises the sequence of SEQ ID NO:1 or a variant sequence having at least 90% sequence identity thereto or a fragment of any of these sequences.

## Patentansprüche

1. Verfahren zur Bestimmung, ob ein Subjekt an Krebs leidet oder anfällig für die Entwicklung von Krebs ist, bei dem man
(a) eine Blutprobe von einem Subjekt bereitstellt;
(b) das Vorhandensein von Antikörpern gegen ein Thrombospondin Typ-1-Domäne-enthaltendes 7A (THSD7A)-Protein in der Blutprobe bestimmt;
wobei das Vorhandensein von Antikörpern gegen das THSD7A-Protein anzeigt, dass das Subjekt an Krebs leidet oder anfällig für die Entwicklung von Krebs ist.

2. Verfahren nach Anspruch 1, bei dem das THSD7A-Protein die Sequenz von SEQ ID NO:1 oder eine Sequenz mit mindestens 90% Sequenzidentität dazu oder ein Fragment von einer dieser Sequenzen umfasst.

3. Verfahren nach Anspruch 1 oder 2, bei dem das Subjekt, von dem angenommen wird, dass es an Krebs leidet, eine membranöse Nephropathie aufweist.

4. Verfahren nach den Ansprüchen 1-3, wobei der Krebs ausgewählt ist aus Nieren-, Prostata-, Schilddrüsen-, Blasen-, Ösophagus-, Dickdarm- oder Brustkrebs, jedoch nicht darauf beschränkt ist.

5. Verfahren zum Screening von Wirkstoffkandidaten zur Identifizierung eines Wirkstoffs, der für die Behandlung von Krebs geeignet ist, bei dem man:
(a) eine Zelle bereitstellt, die ein Thrombospondin Typ-1-Domäne-enthaltendes 7A (THSD7A)-Protein exprimiert;
(b) der Zelle einen Wirkstoffkandidaten zugibt; und
(c) die Wirkung des Wirkstoffkandidaten auf die Expression der Sequenz oder des Sequenz-Fragments oder auf Wachstum, Überleben oder Motilität der Zelle bestimmt;
wobei die Verringerung der Expression oder die Verringerung von Wachstum, Überleben oder Motilität der Zelle anzeigt, dass der Wirkstoffkandidat als Wirkstoff für die Behandlung von Krebs geeignet sein kann.

6. Verfahren nach Anspruch 5, bei dem das Thrombospondin Typ-1-Domäne-enthaltendes 7A (THSD7A)-Protein die Sequenz von SEQ ID NO:1 oder eine Sequenz mit mindestens 90% Sequenzidentität dazu oder ein Fragment von einer dieser Sequenzen umfasst oder daraus besteht.

7. Verwendung eines Antikörpers, eines Antikörperfragments, eines Nanobody oder eines Nanobody-Fragments, der/das selektiv an ein Thrombospondin Typ-1-Domäne-enthaltendes 7A (THSD7A)-Protein bindet, für die In-vitro-Diagnose von Krebs.

8. Verwendung eines Antikörpers, eines Antikörperfragments, eines Nanobody oder eines Nanobody-Fragments nach Anspruch 7, bei der das Thrombospondin Typ-1-Domäne-enthaltendes 7A (THSD7A)-Protein die Sequenz von SEQ ID NO:1 oder eine Sequenzvariante mit mindestens 90 % Sequenzidentität dazu oder ein Fragment von einer dieser Sequenzen umfasst.

## Revendications

1. Procédé pour déterminer si un sujet est atteint d'un cancer ou est susceptible de développer un cancer, comprenant les étapes consistant à
a) fournir un échantillon de sang provenant d'un sujet ;
b) déterminer la présence d'anticorps contre une protéine 7A contenant un domaine de thrombospondine de type 1 (THSD7A) dans ledit échantillon de sang ;
dans lequel la présence d'anticorps dirigés contre la protéine THSD74A indique que ledit sujet est atteint d'un cancer ou est susceptible de développer un cancer.

2. Procédé selon la revendication 1, dans lequel la protéine THSD7A comprend la séquence de SEQ ID NO: 1 ou une séquence ayant un degré d'identité de séquence d'au moins 90 % avec celle-ci ou un fragment de l'une quelconque de ces séquences.

3. Procédé selon la revendication 1 ou 2, dans lequel le sujet qui est suspecté de souffrir d'un cancer est atteint de néphropathie membraneuse.

4. Procédé selon les revendications 1 à 3, dans lequel ledit cancer est choisi parmi, mais sans s'y limiter, un cancer du rein, de la prostate, de la thyroïde, de la vessie, de l'œsophage, du côlon ou du sein.

5. Procédé de criblage de candidats de médicament pour identifier un médicament qui soit utile pour le traitement d'un cancer, comprenant les étapes consistant à :
a) fournir une cellule qui exprime une protéine 7A contenant un domaine de thrombospondine de type 1 (THSD7A),
b) ajouter un candidat de médicament à ladite cellule ; et
c) déterminée l'effet dudit candidat de médicament sur l'expression de ladite séquence ou dudit fragment de séquence ou sur la croissance, la survie, ou la motilité de la cellule ;
dans lequel la réduction de l'expression ou la réduction de la croissance, de la survie, ou de la motilité de la cellule indique que le candidat de médicament peut être utile en tant qu'un médicament pour le traitement d'un cancer.

6. Procédé selon la revendication 5, dans lequel la protéine 7A contenant un domaine de thrombospondine de type 1 (THSD7A) comprend ou consiste en la séquence de SEQ ID NO: 1 ou une séquence ayant un degré d'identité de séquence d'au moins 90 % avec celle-ci ou un fragment de l'une quelconque de ces séquences.

7. Utilisation d'un anticorps, fragment d'anticorps, nanocorps ou fragment de nanocorps, qui se lie sélectivement à une protéine 7A contenant un domaine de thrombospondine de type 1 (THSD7A), pour le diagnostic in vitro d'un cancer.

8. Utilisation d'un anticorps, fragment d'anticorps, nanocorps ou fragment de nanocorps selon la revendication 7, dans laquelle ladite protéine 7A contenant un domaine de thrombospondine de type 1 (THSD7A) comprend la séquence de SEQ ID NO: 1 ou une séquence variante ayant un degré d'identité de séquence d'au moins 90 % avec celle-ci ou un fragment de l'une quelconque de ces séquences.


### SEQUENCE LISTING

<110> Universitätsklinikum Hamburg-Eppendorf
<120> NEW TARGET FOR CANCER THERAPY
<130> P 101650
<160> 2
<170> PatentIn version 3.5
<210> 1
   <211> 1657
   <212> PRT
   <213> Homo sapiens
<400> 1
   Met Gly Leu Gln Ala Arg Arg Trp Ala Ser Gly Ser Arg Gly Ala Ala
   1               5                   10                  15
   Gly Pro Arg Arg Gly Val Leu Gln Leu Leu Pro Leu Pro Leu Pro Leu
               20                  25                  30
   Pro Leu Leu Leu Leu Leu Leu Leu Arg Pro Gly Ala Gly Arg Ala Ala
           35                  40                  45
   Ala Gln Gly Glu Ala Glu Ala Pro Thr Leu Tyr Leu Trp Lys Thr Gly
       50                  55                  60
   Pro Trp Gly Arg Cys Met Gly Asp Glu Cys Gly Pro Gly Gly Ile Gln
   65                  70                  75                  80
   Thr Arg Ala Val Trp Cys Ala His Val Glu Gly Trp Thr Thr Leu His
                   85                  90                  95
   Thr Asn Cys Lys Gln Ala Glu Arg Pro Asn Asn Gln Gln Asn Cys Phe
               100                 105                 110
   Lys Val Cys Asp Trp His Lys Glu Leu Tyr Asp Trp Arg Leu Gly Pro
           115                 120                 125
   Trp Asn Gln Cys Gln Pro Val Ile Ser Lys Ser Leu Glu Lys Pro Leu
       130                 135                 140
   Glu Cys Ile Lys Gly Glu Glu Gly Ile Gln Val Arg Glu Ile Ala Cys
   145                 150                 155                 160
   Ile Gln Lys Asp Lys Asp Ile Pro Ala Glu Asp Ile Ile Cys Glu Tyr
                   165                 170                 175
   Phe Glu Pro Lys Pro Leu Leu Glu Gln Ala Cys Leu Ile Pro Cys Gln
               180                 185                 190
   Gln Asp Cys Ile Val Ser Glu Phe Ser Ala Trp Ser Glu Cys Ser Lys
           195                 200                 205
   Thr Cys Gly Ser Gly Leu Gln His Arg Thr Arg His Val Val Ala Pro
       210                 215                 220
   Pro Gln Phe Gly Gly Ser Gly Cys Pro Asn Leu Thr Glu Phe Gln Val
   225                 230                 235                 240
   Cys Gln Ser Ser Pro Cys Glu Ala Glu Glu Leu Arg Tyr Ser Leu His
                   245                 250                 255
   Val Gly Pro Trp Ser Thr Cys Ser Met Pro His Ser Arg Gln Val Arg
               260                 265                 270
   Gln Ala Arg Arg Arg Gly Lys Asn Lys Glu Arg Glu Lys Asp Arg Ser
           275                 280                 285
   Lys Gly Val Lys Asp Pro Glu Ala Arg Glu Leu Ile Lys Lys Lys Arg
       290                 295                 300
   Asn Arg Asn Arg Gln Asn Arg Gln Glu Asn Lys Tyr Trp Asp Ile Gln
   305                 310                 315                 320
   Ile Gly Tyr Gln Thr Arg Glu Val Met Cys Ile Asn Lys Thr Gly Lys
                   325                 330                 335
   Ala Ala Asp Leu Ser Phe Cys Gln Gln Glu Lys Leu Pro Met Thr Phe
               340                 345                 350
   Gln Ser Cys Val Ile Thr Lys Glu Cys Gln Val Ser Glu Trp Ser Glu
           355                 360                 365
   Trp Ser Pro Cys Ser Lys Thr Cys His Asp Met Val Ser Pro Ala Gly
       370                 375                 380
   Thr Arg Val Arg Thr Arg Thr Ile Arg Gln Phe Pro Ile Gly Ser Glu
   385                 390                 395                 400
   Lys Glu Cys Pro Glu Phe Glu Glu Lys Glu Pro Cys Leu Ser Gln Gly
                   405                 410                 415
   Asp Gly Val Val Pro Cys Ala Thr Tyr Gly Trp Arg Thr Thr Glu Trp
               420                 425                 430
   Thr Glu Cys Arg Val Asp Pro Leu Leu Ser Gln Gln Asp Lys Arg Arg
           435                 440                 445
   Gly Asn Gln Thr Ala Leu Cys Gly Gly Gly Ile Gln Thr Arg Glu Val
       450                 455                 460
   Tyr Cys Val Gln Ala Asn Glu Asn Leu Leu Ser Gln Leu Ser Thr His
   465                 470                 475                 480
   Lys Asn Lys Glu Ala Ser Lys Pro Met Asp Leu Lys Leu Cys Thr Gly
                   485                 490                 495
   Pro Ile Pro Asn Thr Thr Gln Leu Cys His Ile Pro Cys Pro Thr Glu
               500                 505                 510
   Cys Glu Val Ser Pro Trp Ser Ala Trp Gly Pro Cys Thr Tyr Glu Asn
           515                 520                 525
   Cys Asn Asp Gln Gln Gly Lys Lys Gly Phe Lys Leu Arg Lys Arg Arg
       530                 535                 540
   Ile Thr Asn Glu Pro Thr Gly Gly Ser Gly Val Thr Gly Asn Cys Pro
   545                 550                 555                 560
   His Leu Leu Glu Ala Ile Pro Cys Glu Glu Pro Ala Cys Tyr Asp Trp
                   565                 570                 575
   Lys Ala Val Arg Leu Gly Asn Cys Glu Pro Asp Asn Gly Lys Glu Cys
               580                 585                 590
   Gly Pro Gly Thr Gln Val Gln Glu Val Val Cys Ile Asn Ser Asp Gly
           595                 600                 605
   Glu Glu Val Asp Arg Gln Leu Cys Arg Asp Ala Ile Phe Pro Ile Pro
       610                 615                 620
   Val Ala Cys Asp Ala Pro Cys Pro Lys Asp Cys Val Leu Ser Thr Trp
   625                 630                 635                 640
   Ser Thr Trp Ser Ser Cys Ser His Thr Cys Ser Gly Lys Thr Thr Glu
                   645                 650                 655
   Gly Lys Gln Ile Arg Ala Arg Ser Ile Leu Ala Tyr Ala Gly Glu Glu
               660                 665                 670
   Gly Gly Ile Arg Cys Pro Asn Ser Ser Ala Leu Gln Glu Val Arg Ser
           675                 680                 685
   Cys Asn Glu His Pro Cys Thr Val Tyr His Trp Gln Thr Gly Pro Trp
       690                 695                 700
   Gly Gln Cys Ile Glu Asp Thr Ser Val Ser Ser Phe Asn Thr Thr Thr
   705                 710                 715                 720
   Thr Trp Asn Gly Glu Ala Ser Cys Ser Val Gly Met Gln Thr Arg Lys
                   725                 730                 735
   Val Ile Cys Val Arg Val Asn Val Gly Gln Val Gly Pro Lys Lys Cys
               740                 745                 750
   Pro Glu Ser Leu Arg Pro Glu Thr Val Arg Pro Cys Leu Leu Pro Cys
           755                 760                 765
   Lys Lys Asp Cys Ile Val Thr Pro Tyr Ser Asp Trp Thr Ser Cys Pro
       770                 775                 780
   Ser Ser Cys Lys Glu Gly Asp Ser Ser Ile Arg Lys Gln Ser Arg His
   785                 790                 795                 800
   Arg Val Ile Ile Gln Leu Pro Ala Asn Gly Gly Arg Asp Cys Thr Asp
                   805                 810                 815
   Pro Leu Tyr Glu Glu Lys Ala Cys Glu Ala Pro Gln Ala Cys Gln Ser
               820                 825                 830
   Tyr Arg Trp Lys Thr His Lys Trp Arg Arg Cys Gln Leu Val Pro Trp
           835                 840                 845
   Ser Val Gln Gln Asp Ser Pro Gly Ala Gln Glu Gly Cys Gly Pro Gly
       850                 855                 860
   Arg Gln Ala Arg Ala Ile Thr Cys Arg Lys Gln Asp Gly Gly Gln Ala
   865                 870                 875                 880
   Gly Ile His Glu Cys Leu Gln Tyr Ala Gly Pro Val Pro Ala Leu Thr
                   885                 890                 895
   Gln Ala Cys Gln Ile Pro Cys Gln Asp Asp Cys Gln Leu Thr Ser Trp
               900                 905                 910
   Ser Lys Phe Ser Ser Cys Asn Gly Asp Cys Gly Ala Val Arg Thr Arg
           915                 920                 925
   Lys Arg Thr Leu Val Gly Lys Ser Lys Lys Lys Glu Lys Cys Lys Asn
       930                 935                 940
   Ser His Leu Tyr Pro Leu Ile Glu Thr Gln Tyr Cys Pro Cys Asp Lys
   945                 950                 955                 960
   Tyr Asn Ala Gln Pro Val Gly Asn Trp Ser Asp Cys Ile Leu Pro Glu
                   965                 970                 975
   Gly Lys Val Glu Val Leu Leu Gly Met Lys Val Gln Gly Asp Ile Lys
               980                 985                 990
   Glu Cys Gly Gln Gly Tyr Arg Tyr  Gln Ala Met Ala Cys  Tyr Asp Gln
           995                 1000                 1005
   Asn Gly  Arg Leu Val Glu Thr  Ser Arg Cys Asn Ser  His Gly Tyr
       1010                 1015                 1020
   Ile Glu  Glu Ala Cys Ile Ile  Pro Cys Pro Ser Asp  Cys Lys Leu
       1025                 1030                 1035
   Ser Glu  Trp Ser Asn Trp Ser  Arg Cys Ser Lys Ser  Cys Gly Ser
       1040                 1045                 1050
   Gly Val  Lys Val Arg Ser Lys  Trp Leu Arg Glu Lys  Pro Tyr Asn
       1055                 1060                 1065
   Gly Gly  Arg Pro Cys Pro Lys  Leu Asp His Val Asn  Gln Ala Gln
       1070                 1075                 1080
   Val Tyr  Glu Val Val Pro Cys  His Ser Asp Cys Asn  Gln Tyr Leu
       1085                 1090                 1095
   Trp Val  Thr Glu Pro Trp Ser  Ile Cys Lys Val Thr  Phe Val Asn
       1100                 1105                 1110
   Met Arg  Glu Asn Cys Gly Glu  Gly Val Gln Thr Arg  Lys Val Arg
       1115                 1120                 1125
   Cys Met  Gln Asn Thr Ala Asp  Gly Pro Ser Glu His  Val Glu Asp
       1130                 1135                 1140
   Tyr Leu  Cys Asp Pro Glu Glu  Met Pro Leu Gly Ser  Arg Val Cys
       1145                 1150                 1155
   Lys Leu  Pro Cys Pro Glu Asp  Cys Val Ile Ser Glu  Trp Gly Pro
       1160                 1165                 1170
   Trp Thr  Gln Cys Val Leu Pro  Cys Asn Gln Ser Ser  Phe Arg Gln
       1175                 1180                 1185
   Arg Ser  Ala Asp Pro Ile Arg  Gln Pro Ala Asp Glu  Gly Arg Ser
       1190                 1195                 1200
   Cys Pro  Asn Ala Val Glu Lys  Glu Pro Cys Asn Leu  Asn Lys Asn
       1205                 1210                 1215
   Cys Tyr  His Tyr Asp Tyr Asn  Val Thr Asp Trp Ser  Thr Cys Gln
       1220                 1225                 1230
   Leu Ser  Glu Lys Ala Val Cys  Gly Asn Gly Ile Lys  Thr Arg Met
       1235                 1240                 1245
   Leu Asp  Cys Val Arg Ser Asp  Gly Lys Ser Val Asp  Leu Lys Tyr
       1250                 1255                 1260
   Cys Glu  Ala Leu Gly Leu Glu  Lys Asn Trp Gln Met  Asn Thr Ser
       1265                 1270                 1275
   Cys Met  Val Glu Cys Pro Val  Asn Cys Gln Leu Ser  Asp Trp Ser
       1280                 1285                 1290
   Pro Trp  Ser Glu Cys Ser Gln  Thr Cys Gly Leu Thr  Gly Lys Met
       1295                 1300                 1305
   Ile Arg  Arg Arg Thr Val Thr  Gln Pro Phe Gln Gly  Asp Gly Arg
       1310                 1315                 1320
   Pro Cys  Pro Ser Leu Met Asp  Gln Ser Lys Pro Cys  Pro Val Lys
       1325                 1330                 1335
   Pro Cys  Tyr Arg Trp Gln Tyr  Gly Gln Trp Ser Pro  Cys Gln Val
       1340                 1345                 1350
   Gln Glu  Ala Gln Cys Gly Glu  Gly Thr Arg Thr Arg  Asn Ile Ser
       1355                 1360                 1365
   Cys Val  Val Ser Asp Gly Ser  Ala Asp Asp Phe Ser  Lys Val Val
       1370                 1375                 1380
   Asp Glu  Glu Phe Cys Ala Asp  Ile Glu Leu Ile Ile  Asp Gly Asn
       1385                 1390                 1395
   Lys Asn  Met Val Leu Glu Glu  Ser Cys Ser Gln Pro  Cys Pro Gly
       1400                 1405                 1410
   Asp Cys  Tyr Leu Lys Asp Trp  Ser Ser Trp Ser Leu  Cys Gln Leu
       1415                 1420                 1425
   Thr Cys  Val Asn Gly Glu Asp  Leu Gly Phe Gly Gly  Ile Gln Val
       1430                 1435                 1440
   Arg Ser  Arg Pro Val Ile Ile  Gln Glu Leu Glu Asn  Gln His Leu
       1445                 1450                 1455
   Cys Pro  Glu Gln Met Leu Glu  Thr Lys Ser Cys Tyr  Asp Gly Gln
       1460                 1465                 1470
   Cys Tyr  Glu Tyr Lys Trp Met  Ala Ser Ala Trp Lys  Gly Ser Ser
       1475                 1480                 1485
   Arg Thr  Val Trp Cys Gln Arg  Ser Asp Gly Ile Asn  Val Thr Gly
       1490                 1495                 1500
   Gly Cys  Leu Val Met Ser Gln  Pro Asp Ala Asp Arg  Ser Cys Asn
       1505                 1510                 1515
   Pro Pro  Cys Ser Gln Pro His  Ser Tyr Cys Ser Glu  Thr Lys Thr
       1520                 1525                 1530
   Cys His  Cys Glu Glu Gly Tyr  Thr Glu Val Met Ser  Ser Asn Ser
       1535                 1540                 1545
   Thr Leu  Glu Gln Cys Thr Leu  Ile Pro Val Val Val  Leu Pro Thr
       1550                 1555                 1560
   Met Glu  Asp Lys Arg Gly Asp  Val Lys Thr Ser Arg  Ala Val His
       1565                 1570                 1575
   Pro Thr  Gln Pro Ser Ser Asn  Pro Ala Gly Arg Gly  Arg Thr Trp
       1580                 1585                 1590
   Phe Leu  Gln Pro Phe Gly Pro  Asp Gly Arg Leu Lys  Thr Trp Val
       1595                 1600                 1605
   Tyr Gly  Val Ala Ala Gly Ala  Phe Val Leu Leu Ile  Phe Ile Val
       1610                 1615                 1620
   Ser Met  Ile Tyr Leu Ala Cys  Lys Lys Pro Lys Lys  Pro Gln Arg
       1625                 1630                 1635
   Arg Gln  Asn Asn Arg Leu Lys  Pro Leu Thr Leu Ala  Tyr Asp Gly
       1640                 1645                 1650
   Asp Ala  Asp Met
       1655
<210> 2
   <211> 10607
   <212> DNA
   <213> Homo sapiens
<400> 2
   attctgcgag gaatggcagc ggcagcagca tccccgccag aggcggcggc ggcggcggca      60
   gcggccacgg ccaccaccgc ggacgctatt gttccctggt gttagacgcg ctctccctcc     120
   ttctcatctg aagcgaacaa tagcaggaaa gagctttgct ttctgtactc tttgaaaaga     180
   cgttccaaga gcggagaaaa attccctgcc gagcgtgcta cggctctgga ccctggagtg     240
   gctgcaggcg gcatggggct gcaagccagg cgctgggcgt ccgggagccg gggcgctgcg      300
   gggccgcgcc ggggcgtcct gcagctgctg ccgctgccgc tgccgctgcc gctgctcctg     360
   ctgctgctgc tacgcccggg cgccggcagg gctgcggcgc agggcgaggc ggaggcgccc     420
   accctctatc tgtggaagac tggtccatgg ggccgatgta tgggagatga atgtggtccc     480
   ggaggcatcc aaacgagggc tgtgtggtgt gctcatgtgg agggatggac tacactgcat     540
   actaactgta agcaggccga gagacccaat aaccagcaga attgtttcaa agtttgcgat     600
   tggcacaaag agttgtacga ctggagactg ggaccttgga atcagtgtca gcccgtgatt     660
   tcaaaaagcc tagagaaacc tcttgagtgc attaaggggg aagaaggtat tcaggtgagg     720
   gagatagcgt gcatccagaa agacaaagac attcctgcgg aggatatcat ctgtgagtac     780
   tttgagccca agcctctcct ggagcaggct tgcctcattc cttgccagca agattgcatc     840
   gtgtctgaat tttctgcctg gtccgaatgc tccaagacct gcggcagcgg gctccagcac    900
   cggacgcgtc atgtggtggc gcccccgcag ttcggaggct ctggctgtcc aaacctgacg     960
   gagttccagg tgtgccaatc cagtccatgc gaggccgagg agctcaggta cagcctgcat    1020
   gtggggccct ggagcacctg ctcaatgccc cactcccgac aagtaagaca agcaaggaga    1080
   cgcgggaaga ataaagaacg ggaaaaggac cgcagcaaag gagtaaagga tccagaagcc    1140
   cgcgagctta ttaagaaaaa gagaaacaga aacaggcaga acagacaaga gaacaaatat    1200
   tgggacatcc agattggata tcagaccaga gaggttatgt gcattaacaa gacggggaaa   1260
   gctgctgatt taagcttttg ccagcaagag aagcttccaa tgaccttcca gtcctgtgtg    1320
   atcaccaaag agtgccaggt ttccgagtgg tcagagtgga gcccctgctc aaaaacatgc    1380
   catgacatgg tgtcccctgc aggcactcgt gtaaggacac gaaccatcag gcagtttccc    1440
   attggcagtg aaaaggagtg tccagaattt gaagaaaaag aaccctgttt gtctcaagga    1500
   gatggagttg tcccctgtgc cacgtatggc tggagaacta cagagtggac tgagtgccgt    1560
   gtggaccctt tgctcagtca gcaggacaag aggcgcggca accagacggc cctctgtgga 1620
   gggggcatcc agacccgaga ggtgtactgc gtgcaggcca acgaaaacct cctctcacaa 1680
   ttaagtaccc acaagaacaa agaagcctca aagccaatgg acttaaaatt atgcactgga 1740
   cctatcccta atactacaca gctgtgccac attccttgtc caactgaatg tgaagtttca 1800
   ccttggtcag cttggggacc ttgtacttat gaaaactgta atgatcagca agggaaaaaa 1860
   ggcttcaaac tgaggaagcg gcgcattacc aatgagccca ctggaggctc tggggtaacc 1920
   ggaaactgcc ctcacttact ggaagccatt ccctgtgaag agcctgcctg ttatgactgg 1980
   aaagcagtga gactgggaaa ctgcgagcca gataacggaa aggagtgtgg tccaggcacg 2040
   caagttcaag aggttgtgtg catcaacagt gatggagaag aagttgacag acagctgtgc 2100
   agagatgcca tcttccccat ccctgtggcc tgtgatgccc catgcccgaa agactgtgtg 2160
   ctcagcacat ggtctacgtg gtcctcctgc tcacacacct gctcagggaa aacgacagaa 2220
   gggaaacaga tacgagcacg atccattctg gcctatgcgg gtgaagaagg tggaattcgc 2280
   tgtccaaata gcagtgcttt gcaagaagta cgaagctgta atgagcatcc ttgcacagtg 2340
   taccactggc aaactggtcc ctggggccag tgcattgagg acacctcagt atcgtccttc 2400
   aacacaacta cgacttggaa tggggaggcc tcctgctctg tcggcatgca gacaagaaaa 2460
   gtcatctgtg tgcgagtcaa tgtgggccaa gtgggaccca aaaaatgtcc tgaaagcctt 2520
   cgacctgaaa ctgtaaggcc ttgtctgctt ccttgtaaga aggactgtat tgtgacccca 2580
   tatagtgact ggacatcatg cccctcttcg tgtaaagaag gggactccag tatcaggaag 2640
   cagtctaggc atcgggtcat cattcagctg ccagccaacg ggggccgaga ctgcacagat 2700
   cccctctatg aagagaaggc ctgtgaggca cctcaagcgt gccaaagcta caggtggaag 2760
   actcacaaat ggcgcagatg ccaattagtc ccttggagcg tgcaacaaga cagccctgga 2820
   gcacaggaag gctgtgggcc tgggcgacag gcaagagcca ttacttgtcg caagcaagat 2880
   ggaggacagg ctggaatcca tgagtgccta cagtatgcag gccctgtgcc agcccttacc 2940
   caggcctgcc agatcccctg ccaggatgac tgtcaattga ccagctggtc caagttttct 3000
   tcatgcaatg gagactgtgg tgcagttagg accagaaagc gcactcttgt tggaaaaagt 3060
   aaaaagaagg aaaaatgtaa aaattcccat ttgtatcccc tgattgagac tcagtattgt 3120
   ccttgtgaca aatataatgc acaacctgtg gggaactggt cagactgtat tttaccagag 3180
   ggaaaagtgg aagtgttgct gggaatgaaa gtacaaggag acatcaagga atgcggacaa 3240
   ggatatcgtt accaagcaat ggcatgctac gatcaaaatg gcaggcttgt ggaaacatct 3300
   agatgtaaca gccatggtta cattgaggag gcctgcatca tcccctgccc ctcagactgc 3360
   aagctcagtg agtggtccaa ctggtcgcgc tgcagcaagt cctgtgggag tggtgtgaag 3420
   gttcgttcta aatggctgcg tgaaaaacca tataatggag gaaggccttg ccccaaactg 3480
   gaccatgtca accaggcaca ggtgtatgag gttgtcccat gccacagtga ctgcaaccag 3540
   tacctatggg tcacagagcc ctggagcatc tgcaaggtga cctttgtgaa tatgcgggag 3600
   aactgtggag agggcgtgca aacccgaaaa gtgagatgca tgcagaatac agcagatggc 3660
   ccttctgaac atgtagagga ttacctctgt gacccagaag agatgcccct gggctctaga 3720
   gtgtgcaaat taccatgccc tgaggactgt gtgatatctg aatggggtcc atggacccaa 3780
   tgtgttttgc cttgcaatca aagcagtttc cggcaaaggt cagctgatcc catcagacaa 3840
   ccagctgatg aaggaagatc ttgccctaat gctgttgaga aagaaccctg taacctgaac 3900
   aaaaactgct accactatga ttataatgta acagactgga gtacatgtca gctgagtgag 3960
   aaggcagttt gtggaaatgg aataaaaaca aggatgttgg attgtgttcg aagtgatggc 4020
   aagtcagttg acctgaaata ttgtgaagcg cttggcttgg agaagaactg gcagatgaac 4080
   acgtcctgca tggtggaatg ccctgtgaac tgtcagcttt ctgattggtc tccttggtca 4140
   gaatgttctc aaacatgtgg cctcacagga aaaatgatcc gaagacgaac agtgacccag 4200
   ccctttcaag gtgatggaag accatgccct tccctgatgg accagtccaa accctgccca 4260
   gtgaagcctt gttatcggtg gcaatatggc cagtggtctc catgccaagt gcaggaggcc 4320
   cagtgtggag aagggaccag aacaaggaac atttcttgtg tagtaagtga tgggtcagct 4380
   gatgatttca gcaaagtggt ggatgaggaa ttctgtgctg acattgaact cattatagat 4440
   ggtaataaaa atatggttct ggaggaatcc tgcagccagc cttgcccagg tgactgttat 4500
   ttgaaggact ggtcttcctg gagcctgtgt cagctgacct gtgtgaatgg tgaggatcta 4560
   ggctttggtg gaatacaggt cagatccaga ccggtgatta tacaagaact agagaatcag 4620
   catctgtgcc cagagcagat gttagaaaca aaatcatgtt atgatggaca gtgctatgaa 4680
   tataaatgga tggccagtgc ttggaagggc tcttcccgaa cagtgtggtg tcaaaggtca 4740
   gatggtataa atgtaacagg gggctgcttg gtgatgagcc agcctgatgc cgacaggtct 4800
   tgtaacccac cgtgtagtca accccactcg tactgtagcg agacaaaaac atgccattgt 4860
   gaagaagggt acactgaagt catgtcttct aacagcaccc ttgagcaatg cacacttatc 4920
   cccgtggtgg tattacccac catggaggac aaaagaggag atgtgaaaac cagtcgggct 4980
   gtacatccaa cccaaccctc cagtaaccca gcaggacggg gaaggacctg gtttctacag 5040
   ccatttgggc cagatgggag actaaagacc tgggtttacg gtgtagcagc tggggcattt 5100
   gtgttactca tctttattgt ctccatgatt tatctagctt gcaaaaagcc aaagaaaccc 5160
   caaagaaggc aaaacaaccg actgaaacct ttaaccttag cctatgatgg agatgccgac 5220
   atgtaacata taacttttcc tggcaacaac cagtttcggc tttctgactt catagatgtc 5280
   cagaggccac aacaaatgta tccaaactgt gtggattaaa atatatttta atttttaaaa 5340
   atggcatcat aaagacaaga gtgaaaatca tactgccact ggagatattt aagacagtac 5400
   cacttatata cagaccatca accgtgagaa ttataggaga tttagctgaa tacatgctgc 5460
   attctgaaag ttttatgtca tcttttctga aatctaccga ctgaaaaacc actttcatct 5520
   ctaaaaaata atggtggaat tggccagtta ggatgcctga tacaagaccg tctgcagtgt 5580
   taatccataa aacttcctag catgaagagt ttctaccaag atctccacaa tactatggtc 5640
   aaattaacat gtgtactcag ttgaatgaca cacattatgt cagattatgt acttgctaat 5700
   aagcaatttt aacaatgcat aacaaataaa ctctaagcta agcagaaaat ccactgaata 5760
   aattcagcat cttggtggtc gatggtagat tttattgacc tgcatttcag agacaaagcc 5820
   tcttttttaa gacttcttgt ctctctccaa agtaagaatg ctggacaagt actagtgtct 5880
   tagaagaacg agtcctcaag ttcagtattt tatagtggta attgtctgga aaactaattt 5940
   acttgtgtta atacaatacg tttctacttt ccctgatttt caaactggtt gcctgcatct 6000
   tttttgctat atggaaggca catttttgca ctatattagt gcagcacgat aggcgcttaa 6060
   ccagtattgc catagaaact gcctcttttc atgtgggatg aagacatctg tgccaagagt 6120
   ggcatgaaga catttgcaag ttcttgtatc ctgaagagag taaagttcag tttggatggc 6180
   agcaagatga aatcagctat tacacctgct gtacacacac ttcctcatca ctgcagccat 6240
   tgtgaaattg acaacatggc ggtaatttaa gtgttgaagt ccctaacccc ttaaccctct 6300
   aaaaggtgga ttcctctagt tggtttgtaa ttgttctttg aaggctgttt atgactagat 6360
   ttttatattt gttatctttg ttaagaaaaa aaaagaaaaa ggaactggat gtctttttaa 6420
   ttttgagcag atggagaaaa taaataatgt atcaatgacc tttgtaacta aaggaaatca 6480
   aaattatatg ttgatttttc tttctctctg atttcccagt ttcagattga atgtctgtct 6540
   tgcaggcagt tatttcaaaa tccatagtct tttgcctttc tcactggcaa aatttgaagc 6600
   aatctctctc ttcaatgtat gatttcaaaa ctaaaatttt aaaagagaag aataaatatc 6660
   ttatccaaga cttcattgca tatctagagc aagagtggca aacttttctt gtaaaggact 6720
   atatagaaaa tattttccac aaggtctatg ttgcaacttc tcaactttgc cattctgcta 6780
   atgcaatctt agtaaataaa agaatgaaca taactgtttt cctatataac ttcccttaca 6840
   aaaacagcca gtaggccacc tttgcaggtc atattttgct gacccctggt ctggatgttt 6900
   tttaatgcta gctctccatg gtgtcatctt tcgttttcat tcatttttca aaggtgagaa 6960
   tttacagtca ttacatttcc cactgtatca gaagtagctg tattttctac agatgtagcc 7020
   ctgggaatgg cacagtggaa caatatttaa ctaactcagc agacttcttg caaatgaaaa 7080
   gaaaactcaa aaggtgccat taaaaagcaa taccattttc aggcaacatt attgcaacaa 7140
   cctttgtgag agaggacact agcttttcag gatatttgaa aaataatata actaacatgc 7200
   tattttatct taaacagctg tgttcctgta aaagtgtttt ggattagaag gtatttttac 7260
   cttcatactg gagtatgaac tccccctgct gttcattaga gtatttgcaa agattacaaa 7320
   agacatcagg ttaggatgtc attgtcccca aaatgaaaat atttgtatgt atttcaataa 7380
   gaactaaatg cagtaagcgc taattcctca ttcaatattc actatattag gaaatgtaaa 7440
   tcatttcaaa gagaacaggt agtcaggaag atgaaaattt tactttaaag caaggattta 7500
   aacaaagaaa accataaaaa caaagtccat tttatctgtt ttattaaaga ggaagaaaaa 7560
   ataatattgt tactttaaaa ctgtgattgt caatatatat aatcaggggt cttccagata 7620
   atcctaaagt tgatctcttt gaataaaata aactccattt gtccaaaata gttaagaaag 7680
   actctattgt ggttacttga aaaattcagc taaccctctt atgaaatgtg tgcaacacct 7740
   attaattttg aaatgtggga tagaatacct actaaagcta tatttaaggt attgtaaaat 7800
   gtcattaatt cagaaaagat tagtatcatc ctacctataa gaattaaatt agcaaatcaa 7860
   aattatagaa tgttatgaaa aatacatttt attcataaat taattagatt ttacagcata 7920
   tatgcatgca tatatatata tacacacaca cacacacaca cccatatata tgagagagag 7980
   gaaattaggt tcaacatgac ctaatattag agaacatatt ggaaggtcac tgtttgttag 8040
   gataaataaa tactggataa cctcgttaga aaggtggaaa gaggagaacg tagcaatctg 8100
   gctattttgg acaaagggaa ggttattata ttcatgttta taacatgaaa atggccttat 8160
   ttttgtttct gacatgagac aataatgagt taaatatttt attggaattt catcaaaacc 8220
   ttatttttac tgttccttct aaaaatttgg gaggggaata tagtagtaac ttctctattg 8280
   acacttttac ctcaccgagg gggtaaaaga tgtagaactg ctgttcccta gaatgaaggt 8340
   ctgttgtttg gtttttaaaa aaagaagagc agaaaaagtt tgacacagta ttatatgatt 8400
   ttgctggact atttcactag aaaccacgta gaataggact aactgatctc ttttgaaagg 8460
   ggctgatttg cttattcatc aataactaat ccaatatgtg gacagtaatc ttaatttcaa 8520
   agcctttttt gaggatacta cggtgtagct taagtgaatt taaaaagagt gcataaaagc 8580
   ttgtttttaa ttacttataa caaagcacaa attacttcta acaaggcaaa tatttattga 8640
   catataagac attctgctag gcattgtaaa aaaaaaaaaa aaagtaatgg cttttgtata 8700
   cataggtact taagacagac atccaaaaga aggaaaaaat gcttaatgag tgaagatagc 8760
   actgaaacat gttgactttt actctaggtt tgtttttaga tggggttagt attttcccct 8820
   acgtaatgta catgtcttta ggccacagta ttaggagttt gatttttccc tgccccaccc 8880
   tcaggtaggt ggcagtggtc attgtagctt aatcagaccc ctgttaagtt cctgggcatt 8940
   tcaaggtaaa ttcacgtctt ccttctcaca gaaattgaat agacctctca cacacttatt 9000
   tacctccccc aacaacctgt atccttggcc ctttctgatt ttctctgctt ggtctctttc 9060
   tactcttgac agctattctt acctacttcc cactaaacat gcccaatttt ttaattaaaa 9120
   aaaaaagaat tcctatattt ccttccctat tagaatcaaa gtgtcactca ctcagagtgt     9180
   gaacatcctg atttacacca aatccactgg tcaaagaaat gaataaaggg aaaaggaaaa     9240
   atgtggaagg catgcaggaa aaaaaaaaaa aaaaaagccc ttttctgtaa aaatgtccat     9300
   gcctacactt gctttagacc atcccatatc ctcctgcctc catctcgagt ctttgttcac     9360
   attccacacc acaagacagt gagggaggac tgtacttttc agtaccatac agaagggaga     9420
   gcctgttcta aagctgacgt gggtctcaat agtacatgag aagcacaatt cccaaatgat     9480
   ctgggatctc ctgtttataa atatattacc aaattcaagt gtgggttctt cttctatttc     9540
   cactgtaaga ttgagggtgt tgaaaaaaca gtagtccttg agcaaatctg tgaggaatct     9600
   tgtcagaatg tgatctttcc tgggactagt ttagttgtga gaactacatc tgcttgttct     9660
   gggaataagg gtctccccag gtcctgccct caggtggccc tggctgcagg acaggatttc     9720
   aggtatcaga aactggtgtg tgagccaagc aaatagttat ccatttataa aaaggatggt     9780
   ttgcagtgtg ttataataat tttatatcta gcagggacaa tgaaaaatgg ttatgtctac     9840
   attattaaat gaatgaaact aatgctaaac agttaatttt gcatttttat ttttctgtgt     9900
   ttctaccaag tcagatctta aaaatgggca gtatatgaag agctttagtt tgtgggagtc     9960
   atgttttcag tgatcaaaaa tctctatatt atttagaata taattgtcag tcttgttgtg    10020
   ttttgtagat ttattgtttt taagccatag tttctagatt acctactttt aaccctcaag    10080
   taaacttttc cctatttttc agaaatctgt ttgtgtttat ttttagaaag taaacattta    10140
   gattcctttt ctggtacttt ttttctccat ttctatcaca cagatttgaa gagatcagta    10200
   tctatcattg taaaatataa ctttattaaa atttgcaatc tgtctttcta taaattatta    10260
   ttccctaaat gtgtatttta tgttaattta actacctgaa tatttatttt gtttaaataa    10320
   ggaggcagct gtctaagaat ttttgttaat attactttgt aaattgcgta atgtaattta    10380
   ttttacatta tgtacatttc ctttttaacg cacaaaatgt ctatgtacaa ttaactacag    10440
   atttgcaaat aatgtcacta agctttattc agtcaataca gatggcatgt gaagatgtaa    10500
   tatgcttttt tacattttca tatgggttat ttgtaaataa ctatattgct gacaaacata    10560
   aatacggaaa tcattttcat tctaaaaaaa aaaaaaaaaa aaaaaaa                  10607